# EUROPÄISCHE PATENTSCHRIFT

(11) **EP 0 972 529 B1**
(45) Veröffentlichungstag und Bekanntmachung des Hinweises auf die Patenterteilung: **23.03.2005**
(21) Anmeldenummer: 99112993.3
(22) Anmeldetag: 06.07.1999
(51) Int. Cl.: A61G 9/02, A61L 2/07

(54) **Maschine zum Reinigen und Desinfizieren von Pflegegeschirr**
Device for washing and disinfecting medical ware
Machine à laver et désinfecter des ustensiles sanitaires

(30) Priorität: 16.07.1998 DE 19831950
(43) Veröffentlichungstag der Anmeldung: 19.01.2000
(73) Patentinhaber: MEIKO Maschinenbau GmbH & Co., 77610 Offenburg (DE)
(72) Erfinder: Lehmann, Denis, Dipl.-Ing.(FH), 77799 Ortenberg (DE); Gaus, Bruno, Dipl.-Ing.(FH), 77654 Offenburg (DE); Näger, Thomas, Dipl.-Ing.(FH), 77652 Offenburg (DE); Kern, Rainer, Dipl.-Ing.(FH), 77654 Offenburg (DE)
(74) Vertreter: Baumann, Eduard, Dipl.-Phys.

(56) Entgegenhaltungen:
- EP-A- 0 870 511
- WO-A-97/20493
- DE-A- 19 509 877
- FR-A- 1 565 407
- US-A- 5 223 229

## Beschreibung

Die Erfindung bezieht sich auf eine Maschine gemäß dem Oberbegriff des Anspruches 1. Derartige Maschinen finden in Krankenanstalten, Pflegeheimen und dergleichen Anwendung. Hier kommt es neben einer bequemen Handhabung und einer raschen Arbeitsweise auf gründliche Sauberkeit und Desinfektion, das heißt auf Hygiene, besonders an.

Eine derartige Maschine ist beispielsweise ein sanitärer Wasserspülapparat für Spülgut wie Steckbecken, Nachtstuhleimer, Urinflaschen. Während zum Reinigen Warmwasser einer bestimmten Temperatur, im Idealfall etwa 55 bis 60° Celsius, über Düsen eingespritzt wird, wird das Spülgut anschließend durch Heißdampf desinfiziert. Im DE-Gbm 7900660 wird ein derartiger sanitärer Wasserspülapparat beschrieben. Hierbei gelangt das zugeführte Frischwasser über eine Salzsole und einen Basenaustauschenthärter in einen Dampferzeuger mit Heizspiralen. Der entstehende Dampf wird über entsprechende Düsen in die Reinigungskammer eingeführt, nachdem der eigentliche Reinigungs-Spülgang beendet ist. Die EP 0670708B1 beschreibt eine Vorrichtung zum Spülen und/oder Desinfizieren von Pflegegeschirr mit einer Reinigungskammer, einem Wasservorratsbehälter mit einer Decke, sowie einem mit dem Wasserleitungsnetz verbindbaren Zulauf. Zur Desinfektion ist entweder eine dezentrale chemische Einrichtung oder ein dezentraler Dampferzeuger vorgesehen. Dieser könnte gegebenenfalls ebenfalls aus dem Vorrat des Wasservorratsbehälters gespeist werden, der Dampf nach beendigtem Spülvorgang mit Wasser zur Desinfektion in die Spülkammer geleitet werden.

Eine Maschine gemäß dem Oberbegriff von Anspruch 1 ist aus der WO-A-97/20493 bekannt.

Der Erfindung liegt die Aufgabe zugrunde, eine Maschine zum Reinigen und Desinfizieren von Pflegegeschirr so auszugestalten, daß die Aufheizzeit des Wassers im Verdampfer, die Verkeimungsgefahr der Anlage und das Volumen verringert werden und die Funktion vereinfacht wird.

Die Aufgabe wird erfindungsgemäß durch die Merkmale des Anspruches 1 gelöst. Weitere Ausgestaltungen der Erfindung sind in den Unteransprüchen unter Schutz gestellt.

Durch den Erfindungsgegenstand soll eine räumlich getrennte Anordnung von Wasserkasten und Dampferzeuger vermieden werden, desgleichen ein Nachfüllen des Dampferzeugers mit Wasser. Die Gefahr eines Verkeimens von Wasserkasten, Pumpund Düsensystem für das Spülwasser soll vermieden werden. Es soll eine ideale Reinigungstemperatur von 55 bis 60° Celsius eingehalten werden, die Aufheizzeiten für das Wasservolumen im Verdampfer sollen reduziert werden, von welchem der erzeugte Wasserdampf zur anschließenden Desinfektion - nach dem Reinigen mit Warmwasser - in die Reinigungskammer gepumpt wird.

Beim Erfindungsgegenstand ist ein Dampferzeuger mit Heizung direkt in den Wasserkasten integriert. Der Dampfbereich ist über eine Trennwand vom Haupt-Wasserkasten getrennt, wobei am Boden eine Wasserverbindung der beiden Bereiche des Wasserkastens verbleibt, eine Dampfsperre jedoch den Übertritt von Dampf in den Haupt-Wasserkasten verhindert. Der erzeugte Dampf im oberen Dampfbereich wird direkt in die Reinigungskammer eingeführt.

Eine Vielzahl von Verbesserungen ermöglicht eine Optimierung dieser Maschine; so eine Steuerung des Wasserzulaufes in den Wasserkasten, Sensoren und steuerbare Ventile für den Wasserzulauf und das Einfüllen in die Reinigungskammer sowie für den Wasserspiegel eine spezielle Anordnung der Dampfzuführung vom Dampfbereich innerhalb des Wasserkastens zum unteren Zentralbereich der Reinigungskammer, von dem der Abfluß wegführt. Weiterhin kann ein Entkalker mit Regenerator bzw. Kalkverhinderer vorgesehen werden.

Statt eines freien Austritts von zugeführtem Frischwasser, in der Regel einer Mischung zwischen Kalt- und Warmwasser, kann auch eine isolierte Zuleitung direkt in einen schmalen Einlaufbehälter am Boden des Wasserkastens erfolgen, von wo es durch Überlauf in einen Dampferzeugerbehälter gelangt, in welchem die Heizeinrichtung vorgesehen ist. Das Flüssigkeitsniveau dieses Dampferzeugerbehälters liegt über dem untersten Wasserspiegel des Wasserkastens.

Ausführungsbeispiele der Erfindung sind in der Zeichnung dargestellt. Es zeigt:
Figur 1 ein erstes Ausführungsbeispiel der Erfindung in schematischer Darstellung,
Figur 2 ein weiteres Ausführungsbeispiel der Erfindung mit unterschiedlichem Wasserzufluß.

Gleiche Teile der Zeichnung sind mit gleichen Bezugszeichen versehen. Dem oben liegenden Wasserkasten wird über ein Warmwasserventil und ein Kaltwasserventil über einen gemeinsamen Zufluß 4 im oberen linken Bereich Wasser zugeführt, wobei ein freier Wasserstrahl vorgesehen ist. Der Wasserkasten ist in einen Haupt-Wasserkasten 1 und einen Dampfbereich 2 unterteilt. Aus dem Hauptwasserkasten 1 wird über eine Pumpvorrichtung 11, steuerbare Magnetventile 12' und Düsen 12 '' Reinigungswasser, dem gegebenenfalls aus einem Reinigungsbehälter 14 Reinigungsmittel zudosiert wird, in die Reinigungskammer 13 eingespritzt. Das Zuführsystem ist allgemein mit 12 bezeichnet. Zum anschließenden Desinfizieren des Spülgutes wird Dampf aus dem Dampfraum 2 über das oben liegende Überlaufventil 5 und eine entsprechende Dampfleitung in die Reinigungskammer 13 eingeführt, von dem aus auch der Abfluß 16 ausgeht. Der maximale Wasserspiegel des Wasserkastens 1 ist mit 9, der minimale Wasserspiegel ist mit 10 bezeichnet.

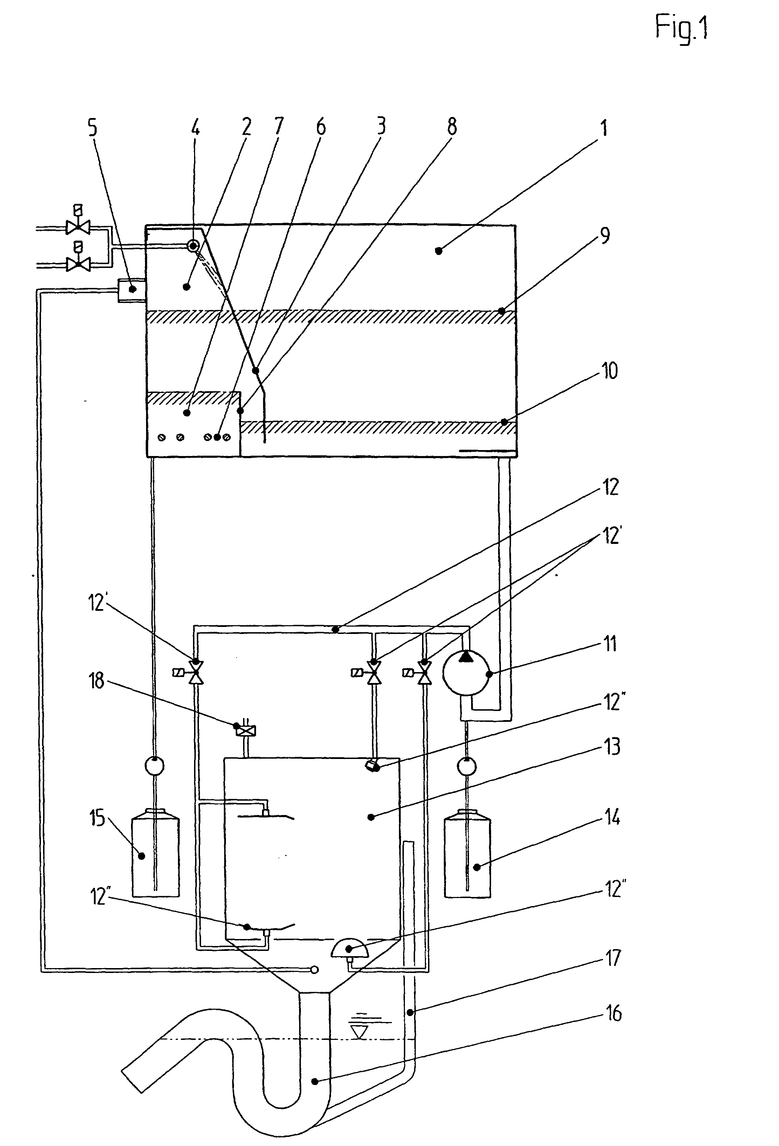

Im unteren Teil von Figur 1 ist weiterhin über einen entsprechenden Behälter 15 ein Kalkverhinderer vorgesehen, der in den Verdampferbehälter 7 eingeführt wird, um ein Verkalken der dort angeordneten Heizeinrichtung 6 zu verhindern.

Die Abtrennung des - kleineren - Dampfbereiches 2 vom Haupt-Wasserkasten 1 erfolgt durch ein im wesentlichen schräg von oben nach unten verlaufendes Schottblech 3, das unterhalb des minimalen Wasserspiegels 10, aber oberhalb des gemeinsamen Kastenbodens endet, um eine Dampfsperre zu erhalten. Die Heizeinrichtung 6 ist in einem Verdampferbehälter 7 links unten vorgesehen. Dieser ist nach oben offen und weist Seitenwände 8 über dem minimalen Wasserspiegel 10 auf.

Schließlich ist am unteren Bogen des Ablaufes 16 ein Überlaufrohr 17 vorgesehen, das ein Überfluten der Reinigungskammer 13 bei Verstopfen des Abflusses verhindern kann. Ein Entlüftungsventil 18 an der Oberseite der Reinigungskammer 13 läßt bei Überdruck diesen direkt ins Freie ab. Andere Möglichkeiten wären die Ableitung über eine Entlüftungsleitung oder in den Abflußkanal.

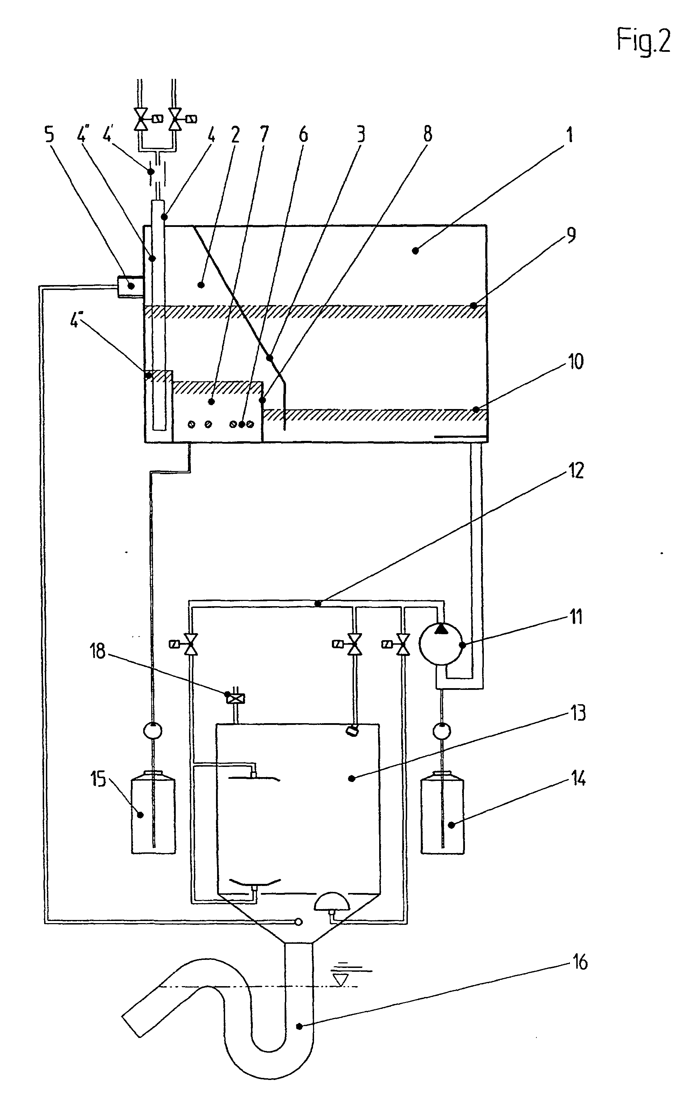

Beim Ausführungsbeispiel von Figur 2 ist im wesentlichen die Zufuhr von Frischwasser etwas anders gelöst. Diese erfolgt über ein direktes Zuleitungsrohr 4, einen Rohrunterbrecher 4' und ein Füllrohr 4'' sowie eine Dampfdrucksperre 4''' in Form eines schmalen Auffangbehälters mit einer gegenüber dem angrenzenden Verdampferbehälter 7 höheren Überlauf-Seitenwand. Bei dieser Ausführung kann ein Sicherheitsüberlauf am Ablauf entfallen. Das Füllwasser strömt über den Rohrunterbrecher in das Füllrohr, die Dampfdrucksperre 4''', die als Wasserbad an der Mündung des Füllrohres erzeugt wird. Dadurch wird verhindert, daß beim Dampferzeugungsvorgang und bei leerem Wasserkasten der Dampf aus dem Rohrunterbrecher austreten kann.

## Patentansprüche

1. Maschine zum Reinigen und Desinfizieren von Pflegegeschirr, das über eine Türe in eine Reinigungskammer eingeführt, dort gehaltert und über Düsen mit Wasser oder Dampf besprüht wird,
mit einem Wasserkasten (1), einem Wasserzulauf, einem Pumpensystem zur Einleitung von Wasser in die Reinigungskammer,
mit einer Heizeinrichtung (6) für die Flüssigkeit und als Dampferzeuger,
mit Zuleitungen, Ableitungen, Verbindungsleitungen, wenigstens einem Überdruck-Entlüftungsventil (18),
gegebenenfalls mit Einrichtungen für chemische Reinigungsmittel, chemische Desinfektionsmittel, Wasserenthärter nebst Regeneriereinrichtung oder Kalkverhinderer,
mit Öffnungs- und Schließventilen für Wasser und Dampf, mit Steuereinrichtungen und Dosiereinrichtungen für das Wasser, den Dampf und gegebenenfalls chemische Reinigungsmittel und chemische Desinfektionsmittel, wobei die Heizeinrichtung (6) als Dampferzeuger in einem Dampfbereich (2) des Wasserkastens angeordnet ist, und wobei der oberhalb des Flüssigkeitspiegels (9, 10) des Dampfbereiches (2) gebildete Dampf durch eine Dampfleitung in die Reinigungskammer (13) geführt wird ,
**dadurch gekennzeichnet,**
**daß** der Dampfbereich (2) im Haupt-Wasserkasten (1) vom Wasserbereich bis unterhalb des niedrigsten Wasserspiegels (10) unter Aufrechterhaltung einer Wasserverbindung und unter Bildung einer Dampfsperre durch eine Trenneinrichtung (3) abgetrennt ist.

2. Maschine nach Anspruch 1, **dadurch gekennzeichnet, daß** der Dampfbereich (2) im Bodenbereich des Wasserzulaufes (4) des Wasserkastens angeordnet ist.

3. Maschine nach Anspruch 2, **dadurch gekennzeichnet, daß** der Wasserspiegel (9, 10) durch Sensoren und Magnetventile in den Wasserzuleitungen (warm/kalt) gesteuert ist.

4. Maschine nach einem der Ansprüche 1 bis 3, **dadurch gekennzeichnet, daß** die Trenneinrichtung ein Schottblech (3) ist, das sich nach unten erstreckt und den Heizbereich (2) neben dem oben liegenden Wassereinlaß (4) bis unterhalb des untersten Flüssigkeitspiegels (10) vom Haupt-Wasserkasten (1) abtrennt, wobei eine untere Wasserverbindung verbleibt, und das zur Wartung einfach entfernbar ist.

5. Maschine nach einem der Ansprüche 1 bis 4, **dadurch gekennzeichnet, daß** die Heizeinrichtung (6) in einem nach oben offenen Verdampferbehälter (7) seitlich am Boden des Wasserkastens angeordnet ist.

6. Maschine nach Anspruch 5, **gekennzeichnet durch** einen mit dem Verdampferbehälter (7) verbundenen Behälter (15) mit einem Kalkverhinderer.

7. Maschine nach einem der Ansprüche 1 bis 6, **dadurch gekennzeichnet, daß** die Dampfleitung vom Überlauf (5) des Wasserkastens (1, 2) ausgeht, der im oberen Dampfbereich (2) angeordnet ist.

8. Maschine nach einem der Ansprüche 1 bis 7, **dadurch gekennzeichnet, daß** das warme/kalte Wasser aus dem Haupt-Wasserkasten (1) über ein Zuführungssystem (12) mit Pumpeinrichtung (11), Ventilen (12') und Einspritzdüsen (12'') in die Reinigungskammer (13) eingeleitet wird.

9. Maschine nach Anspruch 8, **gekennzeichnet durch** steuerbare Magnetventile (12') für die Düsen (12'') zur Anpassung des Zuflusses an den jeweiligen Wasserbedarf.

10. Maschine nach einem der Ansprüche 1 bis 9, **gekennzeichnet durch** ein in das Saugrohr der Pumpeinrichtung (11) über einen Reinigungsbehälter (14) zudosiertes Reinigungsmittel.

11. Maschine nach einem der Ansprüche 1 bis 10, **dadurch gekennzeichnet, daß** der Haupt-Wasserkasten (1) nebst Pumpeinrichtung (11), Magnetventilen (12') und Einspritzdüsen (12'') getrennt vom Verdampferbehälter (7) entleerbar und öffenbar ausgebildet ist, derart, daß der Dampf auch in dieses Zuleitungssystem eindringen und es desinfizieren kann.

12. Maschine nach einem der Ansprüche 1 bis 11, **gekennzeichnet durch** einen Abfluß-Überlauf (17) am Abfluß (16), vorzugsweise an dessen unterem Ablaufbogen.

13. Maschine nach einem der Ansprüche 1 bis 12, **gekennzeichnet durch** ein Überdruck-Entlüftungsventil (18), vorzugsweise ein Magnetventil, direkt an der Oberseite der Reinigungskammer (13), zum Ablassen des Dampfüberdruckes, das direkt ins Freie führen kann, oder das in eine Abluftleitung oder im Abfluß (16) enden kann.

14. Maschine nach einem der Ansprüche 1 bis 13, **gekennzeichnet durch** eine Verbindungsleitung (4, 4') des Wasserzuflusses in einen getrennten Zuflußbehälter (4'''), von dem aus das Wasser **durch** Überlauf des Dampfbereiches (2) in den angrenzenden Verdampferhälter (7) gelangt.

## Claims

1. A machine for cleaning and disinfecting nursing care utensils that are introduced through a door into a cleansing chamber, held in place there and sprayed with water or steam via nozzles,
said machine comprising a water tank (1), a water inlet, a pump system for feeding water into the cleansing chamber;
comprising a heating device (6) for the liquid, serving as a steam generator; comprising feed pipes, drain pipes, connecting conduits, at least one pressure-relief vent valve (18);
if applicable, having devices for chemical cleansers, chemical disinfectants, water softeners including a regeneration device or lime inhibitor;
comprising opening and closing valves for water and steam, with control and dosing devices for the water, the steam and if applicable chemical
cleansers, wherein said heating device (6) as a steam generator is arranged in a steam area (2) of the water tank, and wherein the steam formed above the liquid level (9, 10) of the steam area (2) is fed through a steam pipe into the cleansing chamber (13),
**characterized in that**
the steam area (2) in the main water tank (1) is separated from the water area by a partition (3) reaching below the lowest water level (10) but maintaining a water connection and forming a steam barrier.

2. The machine according to claim 1, **characterized in that** the steam area (2) is located in the bottom area of the water inlet (4) of the water tank.

3. The machine according to claim 2, **characterized in that** the water level (9, 10) is controlled by sensors and solenoid valves in the water feed pipes (hot/cold).

4. The machine according to any one of claims 1 to 3, **characterized in that** the partition is a sheet metal divider (3) which extends downwards and separates the heating area (2) with the water inlet (4) at the top from the main water tank (1) down to below the lowest liquid level (10), but leaving a lower water connection, and which is easily removable for maintenance.

5. The machine according to any one of claims 1 to 4, **characterized in that** the heating device (6) is arranged at the side on the bottom of the water tank, in a vaporizer container (7) that is open at the top.

6. The machine according to claim 5, **characterized by** a container (15) of lime inhibitor connected to the vaporizer container (7).

7. The machine according to any one of claims 1 to 6, **characterized in that** the steam pipe runs from the overflow (5) of the water tank (1, 2), said overflow being located in the upper steam area (2).

8. The machine according to any one of claims 1 to 7, **characterized in that** the hot/cold water is fed from the main water tank (1) into the cleansing chamber (13) via a feed system (12) having a pumping device (11), valves (12'), and injection nozzles (12").

9. The machine according to claim 8, **characterized by** controllable solenoid valves (12') for the nozzles (12"), for adapting the inflow to the particular water requirement.

10. The machine according to any one of claims 1 to 9, **characterized by** a cleanser metered into the suction pipe of the pumping device (11) via a cleansing container (14).

11. The machine according to any one of claims 1 to 10, **characterized in that** the main water tank (1), in addition to the pumping device (11), solenoid valves (12') and injection nozzles (12"), is designed for being emptied and opened independently of the vaporizer container (7) in such a manner that the steam can also penetrate into and disinfect this feed system.

12. The machine according to any one of claims 1 to 11, **characterized by** a drain overflow (17) at the drain (16), preferably at the lower bend in the drain.

13. The machine according to any one of claims 1 to 12, **characterized by** a pressure-relief vent valve (18), preferably a solenoid valve, directly on the upper side of the cleansing chamber (13), for releasing the steam pressure, which can lead directly into the open discharge into an air outlet duct or end in the drain (16).

14. The machine according to any one of claims 1 to 13, **characterized by** a connecting line (4, 4') of the water inflow into a separate inflow reservoir (4"') from which the water reaches the adjacent vaporizer container (7) through the overflow of the steam area (2).

## Revendications

1. Machine pour nettoyer et désinfecter les ustensiles de soins qui sont introduits par une porte dans une chambre de nettoyage dans laquelle ils sont maintenus en position et aspergés d'eau ou de vapeur par l'intermédiaire de buses,
avec un réservoir d'eau (1), une arrivée d'eau, un système de pompage pour introduire de l'eau dans la chambre de nettoyage,
avec un dispositif de chauffage (6) pour le liquide et en tant que générateur de vapeur,
avec des conduites d'alimentation, des conduites d'évacuation, des conduites de liaison, au moins une soupape de surpression de mise à l'air libre (18),
le cas échéant avec des dispositifs pour des nettoyants chimiques, des désinfectants chimiques, des adoucisseurs d'eau ainsi qu'avec un dispositif de régénération ou un système anti-calcaire,
avec des vannes d'ouverture et de fermeture pour l'eau et la vapeur, avec des dispositifs de commande et de dosage de l'eau, de la vapeur et le cas échéant des nettoyants chimiques et des désinfectants chimiques, le dispositif de chauffage (6) étant disposé dans une zone de vapeur (2) du réservoir d'eau où il tient lieu de générateur de vapeur et la vapeur formée au-dessus du niveau de liquide (9, 10) de la zone de vapeur (2) étant acheminée dans une conduite de vapeur jusque dans la chambre de nettoyage (13),
**caractérisée en ce**
**que** la zone de vapeur (2) dans le réservoir d'eau principal (1) est séparée de la zone d'eau jusqu'en dessous du niveau d'eau (10) le plus bas par un organe de séparation (3), ceci tout en maintenant une liaison hydraulique et tout en formant un pare-vapeur.

2. Machine selon la revendication 1, **caractérisée en ce que** la zone de vapeur (2) est disposée dans la partie du fond de l'arrivée d'eau (4) du réservoir d'eau.

3. Machine selon la revendication 2, **caractérisée en ce que** le niveau d'eau (9, 10) est contrôlé par des capteurs et des électrovannes prévus dans les conduites d'alimentation en eau (chaud/froid).

4. Machine selon l'une quelconque des revendications 1 à 3, **caractérisée en ce que** l'organe de séparation est une cloison en tôle (3) qui s'étend vers le bas et qui sépare du réservoir d'eau principal (1), jusqu'en dessous du niveau de liquide (10) le plus bas, la zone de chauffage (2) adjacente à l'arrivée d'eau (4) située au-dessus, une liaison hydraulique étant maintenue dans la partie inférieure, et laquelle cloison est apte à être enlevée facilement pour l'entretien.

5. Machine selon l'une quelconque des revendications 1 à 4, **caractérisée en ce que** le dispositif de chauffage (6) est disposé sur le côté dans le fond du réservoir d'eau dans un récipient évaporateur (7) ouvert vers le haut.

6. Machine selon la revendication 5, **caractérisée par** un récipient (15) contenant un produit anti-calcaire et relié au récipient évaporateur (7).

7. Machine selon l'une quelconque des revendications 1 à 6, **caractérisée en ce que** la conduite de vapeur part du trop-plein (5) du réservoir d'eau (1, 2) disposé dans la zone de vapeur supérieure (2).

8. Machine selon l'une quelconque des revendications 1 à 7, **caractérisée en ce que** l'eau chaude/froide est acheminée dans un système d'amenée (12) muni d'un système de pompage (11), de soupapes (12') et de buses d'injection (12'') depuis le réservoir d'eau principal (1) jusque dans la chambre de nettoyage (13).

9. Machine selon la revendication 8, **caractérisée par** des électrovannes (12') commandables pour les buses (12'') destinées à adapter, cas par cas, la quantité d'eau amenée au besoin en eau.

10. Machine selon l'une quelconque des revendications 1 à 9, **caractérisée par** une dose de nettoyant rajoutée dans le tuyau d'aspiration du système de pompage (11) par l'intermédiaire d'un récipient de nettoyage (14).

11. Machine selon l'une quelconque des revendications 1 à 10, **caractérisée en ce que** le réservoir d'eau principal (1) avec son système de pompage (11), ses électrovannes (12') et ses buses d'injection (12") est apte à être vidangé et ouvert séparément du récipient évaporateur (7) de manière à autoriser la vapeur à également pénétrer dans ce système d'alimentation et à désinfecter celui-ci.

12. Machine selon l'une quelconque des revendications 1 à 11, **caractérisée par** un trop-plein (17) d'écoulement prévu sur le tuyau d'évacuation (16), de préférence dans sa courbure d'écoulement inférieure.

13. Machine selon l'une quelconque des revendications 1 à 12, **caractérisée par** une soupape de surpression de mise à l'air libre (18), de préférence une électrovanne, prévue directement sur la face supérieure de la chambre de nettoyage (13) et destinée à évacuer la surpression de vapeur, ladite soupape pouvant mener directement à l'air libre ou déboucher dans une conduite de mise à l'air ou dans le tuyau d'évacuation (16).

14. Machine selon l'une quelconque des revendications 1 à 13, **caractérisée par** une conduite de liaison (4, 4') d'amenée de l'eau menant à un récipient d'admission séparé (4''') à partir duquel l'eau gagne le récipient évaporateur (7) adjacent par débordement de la zone de vapeur (2).
